# EUROPÄISCHE PATENTSCHRIFT

(11) **EP 0 891 157 B1**
(45) Veröffentlichungstag und Bekanntmachung des Hinweises auf die Patenterteilung: **14.03.2001**
(21) Anmeldenummer: 97925822.5
(22) Anmeldetag: 04.04.1997
(51) Int. Cl.: A61B 18/24, A61M 25/06

(54) **LASERAPPLIKATIONSSET**
LASER APPLICATOR SET
ENSEMBLE D'APPLICATION D'UN FAISCEAU LASER

(30) Priorität: 04.04.1996 DE 19614780; 16.01.1997 DE 19702898
(43) Veröffentlichungstag der Anmeldung: 20.01.1999
(73) Patentinhaber: Somatex Medizintechnische Instrumente GmbH, 15910 Rietzneuendorf (DE)
(72) Erfinder: VOGL, Thomas, J., Prof. Dr. med., D-61462 Königstein (DE); MACK, Martin, G., Dr., D-65795 Hattersheim (DE); KNIEP, Frank, D-14979 Grossbeeren (DE)
(74) Vertreter: Haschick, Gerald
(86) Internationale Anmeldenummer: DE9700730
(87) Internationale Veröffentlichungsnummer: WO9737601

(56) Entgegenhaltungen:
- EP-A- 0 634 909
- EP-A- 0 673 627
- WO-A-92/03963
- WO-A-93/19680
- WO-A-94/26184
- DE-A- 4 137 983
- US-A- 4 959 063
- US-A- 5 312 392
- US-A- 5 330 490
- US-A- 5 401 270

## Beschreibung

Die Erfindung betrifft ein Laserapplikationsset, dabei handelt es sich um ein interventionelles Applikationsset für die minimal invasive, laserinduzierte Thermotherapie von Weichteiltumoren.

Aus dem Stand der Technik ist bekannt, daß mit der aus einem optischen Lichtwellenleiter austretenden Laserstrahlung, beispielsweise der Strahlung eines Nd : YAG-Lasers bei 1064 nm, unter Verwendung einer optischen Faser aus Quarzglas (PCS-, HCS- oder ALL-SILICA-Typ) als Strahlführungssystem, im biologischen Gewebe radialsymmetrische Koagulationsnekrosen gesetzt werden können. Dazu wird die lichtführende Faser entweder auf das Gewebe aufgesetzt oder in das Gewebe eingestochen. Bei diesem Therapieverfahren nimmt man jedoch den Nachteil in Kauf, daß an der Kontaktstelle zwischen Faserendfläche und Gewebe eine hohe Leistungsdichte auftritt, die abhängig vom Faserdurchmesser oberhalb von einigen 100 mW Lichtleistung bereits zur Karbonisierung der Gewebeoberfläche führt und damit eine weitere Ausbreitung der Strahlung im Gewebe durch die hohe Absorption des Kohlenstoffes verhindert.

Weiterhin ist bekannt, eine Applikationsvorrichtung für Laserstrahlung (DE4137983A1), bei der das dem zu behandelnden Objekt zugewandte Ende des strahlenführenden Lichtwellenleiters in einem stirnseitig für Laserstrahlung transparent verschlossenen, röhrenförmigen Hüllkörper, insbesondere einem im objektseitigen Ende luft- und flüssigkeitsdicht verschlossenen Kunststoffschlauch, angeordnet ist, wobei diese Stirnseite des Hüllkörpers und/oder das Ende des Lichtwellenleiters mit einer ein Streuvolumen aufweisenden Streuvorrichtung für Laserstrahlung versehen ist. Dabei werden auch Mehrkammerschläuche verwendet, um eine eventuelle Kühlung über ein angeschlossenes Verteilerstück zu realisieren.

Bekannt ist ein EP 0 634 909 "Arbeitsschaft für die Photo-Thermo-Therapie", wobei ein optischer Arbeitsschaft mit innenliegendem Lichtwellenleiter, welcher umspült werden kann, in einem Endoskop bekannter Bauart eingeführt wird und somit eine Behandlung eines Tumors möglich ist. Ein Nachteil dieser technischen Lösung ist dadurch gegeben, daß eine Repositionierung des Endoskopes mit beinhaltetem Arbeitsschaft mit Lichtwellenleiter nur mit einer neuen Punktion möglich ist.

Bekannt ist weiterhin eine technische Lösung WO 94/26184 "Verfahren und Vorrichtung zur thermischen Verödung biologischen Gewebes", wobei ein Verfahren zur thermischen Verödung biologischen Gewebes mittels über einen Lichtwellenleiter in das Gewebe eingebrachter Laserstrahlung, wobei die Laserstrahlung über der Austrittsfläche der Strahlung aus dem Lichtwellenleiter zugeordneten Mitteln zerstreut wird und um die Austrittsfläche der Strahlung herum durch Injektion einer die Laserstrahlung im wesentlichen nicht absorbierenden, diese aber streuenden, biokompatiblen, mittel- bis hochviskosen Flüssigkeit innerhalb des Gewebes ein von diesem nicht getrenntes Steufluiddepot gebildet wird, das zur kontrollierten Aufheizung des Gewebes benutzt wird, beschrieben ist. Die Vorrichtung zur Durchführung des Verfahrens, bei dem eine mit einer Laserstrahlungsquelle verbindbare, in einem Lumen einer mehrlumigen Kanüle angeordnete Lichtwellenleiteranordnung und eine mit mindestens einem weiteren Lumen der Kanüle verbundene Vorrichtung zur Zuführung einer mittel- bis hochviskosen Flüssigkeit vorhanden ist, ist dargestellt. Ein Nachteil dieser technischen Lösung ist dadurch gegeben, daß eine Repositionierung des Endoskopes mit beinhaltetem Arbeitsschaft mit Lichtwellenleiter nur mit einer neuen Punktion möglich ist.

Der Nachteil bei all diesen Therapieverfahren, welche diesseitig aufgezeichnet wurden, ist darin begründet, dass die entsprechenden Behandlungsmethoden der einzelnen technischen Lösungen mit ihren Vorrichtungen keine Flexibilität bei der Behandlung von Weichteiltumoren ermöglicht.

Ziel der Erfindung ist es, ein Laserapplikationsset zu realisieren, welches eine schonende Behandlung für den Patienten gewährleistet, und eine Überwachung über den Computertomographen bzw. Magnetresonanztomographen möglich ist.

Aufgabe der Erfindung ist es, ein Laserapplikationsset zu schaffen, welches die Nachteile des Standes der Technik und der bisherigen Therapieverfahren aufhebt, indem ein interventionelles Applikationsset für die Lasertherapie von Weichteiltumoren gefunden wird, wodurch eine genaue Positionierung durch die Stabilität des Laserapplikationssets zu schaffen ist und eine hohe Flexibilität mit dem Einsatz eines Lasers bei der Behandlung von Weichteiltumoren gewährleistet wird.

Die Aufgabe wird erfindungsgemäß dadurch gelöst, daß ein Laserapplikationsset gemäß des Anspruches 1 realisiert wird.

Als ein weiterer Bestandteil des Laserapplikationssets ist ein Hüllkatheter mit einem Innenmandrin vorhanden. Dieser Hüllkatheter mit dem Innenmandrin wird bei Entnahme des Mandrins aus der Einführschleuse eingeführt. Dabei ist der Hüllkatheter so ausgefertigt, daß er am distalen Ende luft- und flüssigkeitsdicht verschlossen ist und eine Hüllkatheterspitze aufweist, welche wesentlich dazu beiträgt, daß eine Repositionierung des Hüllkatheters in dem zu behandelnden Tumor durchgeführt werden kann. Auf dem Hüllkatheter sind ebenfalls Markierungen in 1 cm Abständen aufgebracht, womit eine genaue Positionierung des Hüllkatheters bei Einführung in die Einführschleuse durchgeführt werden kann. Der Beginn der Markierung auf dem Hüllkatheter ist so ausgeführt, daß bei Erreichen der ersten Markierung dieser aus der Einführschleuse etwa 30 mm herausragt, was für die Funktionsfähigkeit des Laserapplikationssets wesentlich ist. Innerhalb des Hüllkatheters ist ein Innenmandrin vorhanden, welcher am Ende ein Griffstück zur Herausnahme dieses aufweist. Der Innenmandrin ist aus einem Edelstahldraht, welcher vorzugsweise aus einem paramagnetischen Werkstoff besteht. Dieser Edelstahldraht ist mit einer Kunststoffhülle umschlossen. Wesentlich ist hierbei, daß durch den Innenmandrin eine Stabilisierung der Einführung des Hüllkatheters in die Einführschleuse hervorgerufen wird und somit eine genaue Positionierung der Hüllkatheterspitze in den zu behandelnden Tumor erfolgen kann. Erfindungswesentlich ist weiterhin, daß die Einführschleuse mit Mandrin sowie der Hüllkatheter mit seinem Innenmandrin aus Werkstoffen bestehen, welche für die Überwachung des Therapieverfahrens über eine Computertomographie bzw. Magnetresonanztomographie anwendbar ist. Vorzugsweise sind die Materialien, aus denen die Einführschleuse und der Mandrin sowie der Hüllkatheter mit dem Innenmandrin bestehen, aus Kunststoffen, wobei dabei PE-, PTFE-, FEP-Kunststoffe verwendet werden.

Das erfindungsgemäße Laserapplikationsset weist folgende Vorteile gegenüber den bekannten Applikationssets für die Lasertherapien für Weichteiltumore auf.

Die auf der Einführschleuse angebrachten Markierungen erlauben eine exakte Positionierung der Einführschleuse am Patienten. Die exakte Lagebeziehung des Laserapplikationssets zur Läsion ist für den Erfolg der Therapie von entscheidender Bedeutung. Weiterhin muß festgestellt werden, daß durch die Membran in dem T-Teil der Einführschleuse eine sichere und rutschfeste Positionierung des erfindungsgemäßen Hüllkatheters erfolgen kann. Der Hüllkatheter ist bis zu 400 °C thermostabil und am distalen Ende luft- und flüssigkeitsdicht verschlossen. Der Hüllkatheter, der somit einen direkten Kontakt des Laserapplikators mit dem Patienten verhindert, verfügt über einen Innenmandrin, bestehend aus einem Draht aus paramagnetischem Werkstoff, der wesentlich zur Stabilisierung des Hüllkatheters beim Einführen beiträgt. Damit kann zum einen der Hüllkatheter dünn gehalten werden, zum anderen wird trotzdem die nötige Stabilität erzielt. Die Markierungen des Hüllkatheters erlauben eine exakte Positionierung des Hüllkatheters und verhindern, daß es zu einem Verschmelzen der Einführschleuse kommt, wenn der lichtdurchlässige Hüllkatheter nicht weit genug aus dem laserlichtundurchlässigen Einführschleusensystem hinausragt. Durch die ständige Kontrolle über den Computertomographen bzw. Magnetresonanztomographen ist eine Repositionierung des Hüllkatheters, welcher innenliegend in der Einführschleuse eingebracht ist, vorhanden. Stellt sich bei Einführung des Laserapplikators ein Defekt oder andere Funktionsstörungen heraus, kann jederzeit ein neuer Laserapplikator eingeführt werden, ohne daß eine neue Therapiephase begonnen werden muß. Durch die entsprechende Materialzusammensetzung ist ein Laserapplikationsset geschaffen, welches kompatibel für die Überwachung mit CT, MRT bzw. Kernspintomographie ist.

Ein weiterer erfindungsgemäßer Vorteil liegt darin begründet, daß bei Beendigung der Lasertherapie und Herausziehen der Einführschleuse über die Schlauchzuführung und dem angeschlossenen Dreiwegehahn behandelnde Mittel, wie zum Beispiel Gewebekleber, eingeführt werden können und somit eine Zellverschleppung des behandelten Tumors untersagt wird, was bisher bei der Lasertherapie von Tumoren einen schwerwiegenden Nachteil darstellte.

Weiterhin ist eine Einmalpunktion eines zu behandelnden Tumors mit dem erfindungsgemäßen Laserapplikationssets gegeben, womit eine Tumorverschleppung verhindert wird.

Als weitere Indikation der Anwendung des erfindungsgemäßen Laserapplikationssets sind folgende Gesichtspunkte zu betrachten. Mit diesem erfindungsgemäßen Laserapplikationsset ist eine perkutane Lasertherapie von beispielsweise Lebermetastasen, die aufgrund ihrer Verteilung nicht operativ entfernbar sind, möglich. Weiterhin ist eine Lasertherapie von operablen Metastasen gegeben, wenn der Patient die Operation ablehnt. Zugleich ist eine Überführung eines Patienten in einen operablen Zustand durch die Laserbehandlung mit Hilfe des Laserapplikationssets von einer oder mehreren Metastasen möglich. Dabei können Metastasen bis maximal 4 cm Durchmesser behandelt werden. Als weitere Anwendungsgebiete des Laserapplikationssets sind zu nennen die paliative Therapie von Rezitivtumuren in der Kopf-Halsregion und perkutane Therapie von Weichteiltumoren im Bereich des Beckens.

Der Hüllkatheter des Laserapplikationssets ist mit einem Anschlußstück ausgeführt. An diesem Anschlußstück ist ein Arretierstück eines Innenhüllkatheters verbunden, wobei der Innenhüllkatheter innerhalb des Hüllkatheters des Laserapplikationssets vorhanden ist. Am distalen Ende des Innenhüllkatheters wird ein Spül- und/oder Kühlmittelaustritt realisiert. Das Anschlußstück des Hüllkatheters und das Arretierstück des Innenhüllkatheters sind über ein Verschlußgewinde an dem Anschlußstück fest miteinander arretierbar. Dabei sind an dem Anschlußstück und an dem Arretierstück jeweils Verbindungen, wobei an den Verbindungen T-Stücke angeordnet sind, vorhanden. Über die T-Stücke wird eine Pumpe für den Kreislauf des Spül- und/oder Kühlmittels angeschlossen. Somit wird über ein T-Stück des Innenhüllkatheters eine Spül- und/oder Kühlflüssigkeit, vorzugsweise sterile isotone Kochsalzlösung, über die Verbindung des Arretierstücks in den Innenhüllkatheter geleitet, wobei der Rückfluß zwischen dem Innenhüllkatheter und dem Hüllkatheter über das Anschlußstück und der Verbindung zum T-Stück ausgeführt ist. Somit wird der gesamte punktierte äußere Gewebebereich durch das Kühlmittel optimal gekühlt.

In dem Arretierstück des Innenhüllkatheters ist eine Membrandichtung angeordnet. Durch diese Membrandichtung wird der Innenmandrin des Laserapplikationssets eingeführt beziehungsweise ausgeführt und weiterhin der Laserapplikator bei einer Therapiebehandlung eingeführt. Eine weitere Membrandichtung im Anschlußstück dient zur Abdichtung bei Einfuhr des Innenhüllkatheters in den Hüllkatheter.

Durch den speziellen Aufbau des Hüllkatheters sind weitere wesentliche Vorteile der Anwendung des Laserapplikationssets gegeben. Mit der Anwendung des Laserapplikationssets gelingt es, unter optimalen Bedingungen Koagulationsnekrosen mit einem maximalen Durchmesser von 20 bis 25 mm zu erzielen. Aufgrund von Wärmeabtransport, der im wesentlichen durch Blutfluß in den Gefäßen und Diffusion im Gewebe bedingt ist, ist es in vivo in der Regel nicht möglich, mit dem Laserapplikationsset ohne den Einsatz des Innenhüllkatheters größere Koagulationsnekrosen als 2 x 2 x 2 cm zu induzieren. Die Mehrzahl der Weichteiltumore ist jedoch bereits bei Diagnosestellung 3 bis 4 cm groß. Um ein Volumen von 4 x 4 x 4 cm zu koagulieren, bei der bisherigen Anwendung des Laserapplikationssets des Hauptpatentes sind acht Laserapplikationen und damit auch acht Punktionen notwendig. Dabei wird jeweils mit einer Laserleistung von zirka 5 Watt über 20 min therapiert.

Wesentlich ist, daß durch die Hüllkatheterausführung die Anwendung mit dem Laserapplikationsset Koagulationsnekrosen von bis zu 4,2 x 4,2 x 4,2 cm mit einer Laserapplikation erreicht werden kann. Dabei werden Laserleistungen von bis zu 30 Watt bis zu 15 min. appliziert. Durch den Hüllkatheter, welcher zur Anwendung in dem Laserapplikationsset gelangt, wird erreicht, daß hohe Laserleistungen nur appliziert werden können, wenn eine Kühlung des unmittelbar an dem Hüllkatheter angrenzenden Gewebe gewährleistet ist und somit eine Gewebekarbonisation oder -vaporisation verhindert wird. Eine Karbonisation oder Vaporisation in dem unmittelbar an dem Hüllkatheter angrenzenden Gewebe verhindert eine weitere Ausbreitung der Koagulationszone, da die gesamte Laserleistung bei der verwendeten Wellenlänge von 1064 nm in der unmittelbaren Hüllkatheterumgebung absorbiert werden würde. Im einzelnen hat der Laser-Applikationskatheter in Verbindung mit dem Hüllkatheter des Laserapplikationssets für den Patienten folgende Vorteile:
1. Um eine 4 x 4 x 4 cm gemessene Läsion zu koagulieren ist mit dem Hüllkatheter in Verbindung mit dem Laserapplikationsset nur noch eine Laserapplikation anstelle von acht Laserapplikationen mit dem Laserapplikationsset nötig.
2. Weiterhin reduziert sich durch die deutliche Verringerung der Zahl der Punktierungen das Risiko von Punktionskomplikationen wie Blutungen, Gefäß- und Nervenverletzungen und Organverletzungen.
3. Da die Zahl der für eine Tumorbehandlung notwendigen Punktionen abnimmt, sinkt auch das Risiko einer Tumorzellverschleppung und damit das Risiko einer Impfmetastase.
4. Der Einsatz des speziellen Hüllkatheters im Zusammenhang mit dem Laserapplikationsset führt ferner zu einer deutlichen Kostenreduktion des Verfahrens, da bei einer 4 x 4 x 4 cm messenden Läsion nicht mehr als acht Punktionskanäle mit Fibrinkleber verschlossen werden müssen, sondern nur noch ein Punktionskanal.
5. Da in der Regel von dem Patienten acht Punktionen in einer Therapiesitzung nicht toleriert werden, mußte die zweite Tumorhälfte meist in einer zweiten Therapiesitzung lasertherapiert werden. Bei Einsatz des Hüllkatheters des Laserapplikationssets kann diese zweite Therapiesitzung entfallen.
6. Bei Läsionen bis zu 4 cm Durchmesser wird durch den Einsatz des Hüllkatheters mit dem Laserapplikationsset eine deutlich größere Wahrscheinlichkeit einer 100%igen Tumorkoagulation erreicht. Dadurch kann durch diesen Einsatz eine deutliche Reduktion der Lokalrezidivrate erzielt werden.

Die vorteilhaften Weiterbildungen der Erfindung sind in den Unteransprüchen gekennzeichnet bzw. werden nachstehend zusammen mit der Beschreibung des bevorzugten Ausführungsbeispiels der Erfindung anhand der Figuren näher dargestellt. Es zeigt:
- Figur 1: Punktionsnadel;
- Figur 2: Edelstahldraht;
- Figur 3: Mandrin-Einführschleuse;
- Figur 4: Einführschleuse mit T-Stück;
- Figur 5: Hüllkatheter;
- Figur 6: Innenmandrin-Hüllkatheter;
- Figur 7: Hüllkatheter mit Kühler;
- Figur 8: Spitze Hüllkatheter mit Kühler.

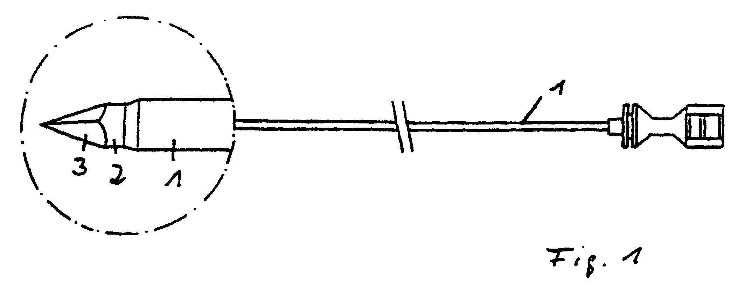

Figur 1 zeigt eine Punktionsnadel 1 mit einem beinhalteten Nadelmandrin 2, welcher eine Nadelmandrinspitze 3 aufweist. Dabei handelt es sich um eine Punktionsnadel bekannter Bauart. Wesentlich ist hierbei, daß die Punktionsnadel 1 aus paramagnetischem Werkstoff besteht und somit eine Anwendung mit dem MRT-Verfahren möglich ist. Die Punktionsnadel 1 weist eine Länge von ca. 200 mm auf und besitzt einen Durchmesser von ca. 1,3 mm. Der Nadelmandrin weist eine Dreikantschliffpunktionsspitze auf, welche über die Punktionsnadel 1 hervorsteht. Mit einem Griffstück kann der Nadelmandrin 2 problemlos aus der Punktionsnadel 1 entfernt werden.

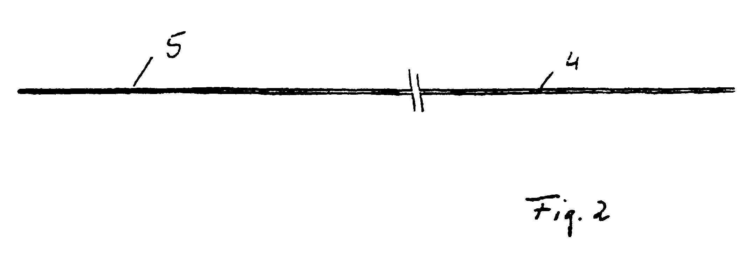

Mit der Figur 2 wird ein Draht 4, welcher aus Edelstahl besteht, dargestellt, wobei eine spezielle Ausführung dieses Drahtes 4 mit einem Federende 5 vorhanden ist, wobei dieses mit einer konisch geschliffenen Feder ausgeführt ist. Die Drahtlänge ist bei dem dargestellten Ausführungsbeispiel etwa 1 Meter, wobei das Federende 5 ca. 100 mm lang ist. Der Draht 4 ist aus paramagnetischem Werkstoff hergestellt.

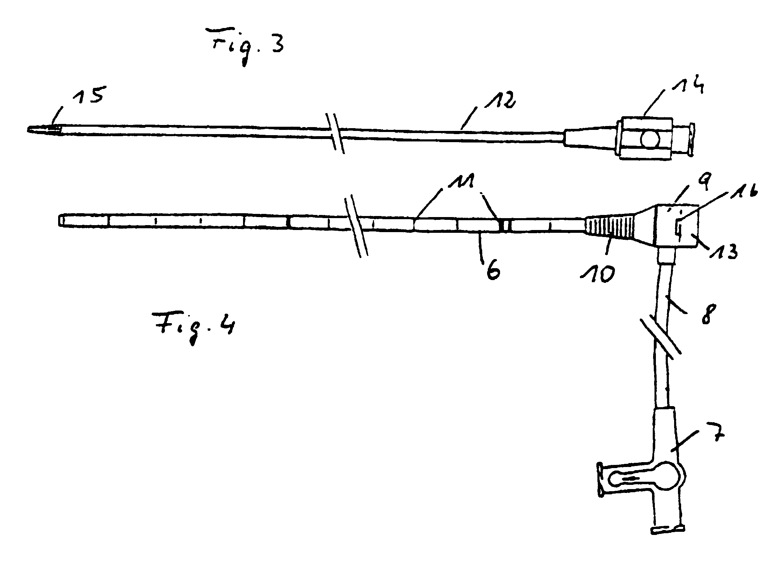

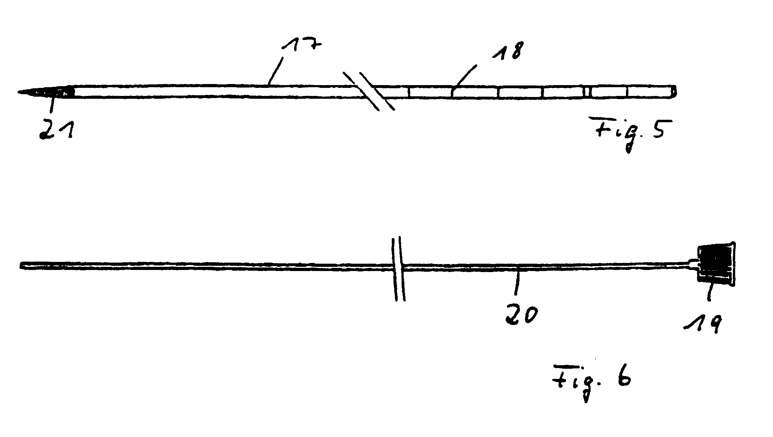

Die Figuren 3 und 4 zeigen die Einführschleuse 6 und den Mandrin 12. Dabei sind die Einführschleuse 6 und der Mandrin 12 aus Kunststoff gefertigt, wie zum Beispiel PE, PTFE, FEP. Die Einführschleuse 6 ist etwa 100 - 200 mm lang und weist einen Durchmesser von ca. 1,5 mm bis - je nach Ausführungsbeispiel - 4 mm auf. Der Mandrin 12, welcher in der Einführschleuse 6 vorhanden ist, ist - je nach Größe der Einführschleuse 6 - paßgerecht ausgeführt. Am proximalen Ende der Einführschleuse 6 ist eine Silikonlamelle 10, woran sich ein T-Stück 9 anschließt, vorhanden. Diese Silikonlamelle 10 ist als eine Silikonmembran in Form einer abknickenden Knicklamelle ausgeführt. Durch die Silikonlamelle 10 wird die Handhabung der Einführschleuse 6 wesentlich verbessert. An dem T-Stück ist eine Schlauchzuführung 8 bzw. anschließend ein Dreiwegehahn 7 angeordnet. Über den Dreiwegehahn 7 und die Schlauchzuführung 8 an das T-Stück 9 ist es möglich, zusätzlich Lokalanasthätikum wie zum Beispiel Gleitmittel, Gewebekleber, Alkohol usw. zu applizieren, was wesentlich dazu beiträgt, daß auch kapselständige Läsionen weitestgehend schmerzfrei therapiert werden können. An dem T-Stück 9 ist weiterhin ein Außengewinde 13 angeordnet, womit der Mandrin 12 mit beinhaltetem Innengewinde in dem Griffstück 14 arretiert werden kann. Zwischen T-Stück 9 und dem anschließenden Außengewinde 13 ist eine Membran 16 vorhanden, welche verhindert, daß Flüssigkeiten austreten können und eine sichere und rutschfeste Positionierung des Hüllkatheters 17 möglich ist. Auf der Einführschleuse 6 sind Markierungen 11 vorhanden. Diese sind vorteilhafterweise im 1 cm Abstand auf der gesamten Einführschleuse 6 angeordnet. Somit ist ein genaues Positionieren der Einführschleuse gegeben. Der Mandrin 12 weist im arretierten Zustand mit der Einführschleuse 6 eine Länge auf, welche sich dadurch unterscheidet, daß etwa 1 cm der Mandrinspitze 15 aus der Einführschleuse 6 am distalen Ende herausragen.

Eine bevorzugte Ausführung ist weiterhin, daß der Mandrin 12 hohl ausgeführt ist. Durch diese hohle Ausführungsform des Mandrins 12 ist eine erhöhte Gleitfähigkeit bei Einführung der Einführschleuse 6 mit dem Mandrin 12 über den Draht 4 an dem Patienten gegeben.

Die Figuren 5 und 6 zeigen die erfindungsgemäßen Hüllkatheter 17 mit Innenmandrin 20. Der Hüllkatheter 17 ist aus einem thermostabilen Kunststoff, welcher bis zu 400 °C Temperaturbelastung standhält. Dabei werden vorteilhafterweise Kunststoffe wie PTFE und FEP angewandt. Der Hüllkatheter 17 weist eine Länge von ca. 400 mm auf. Erfindungswesentlich ist hierbei eine vorhandene Spitze am distalen Ende des Hüllkatheters, welche luft- und flüssigkeitsdicht abgeschlossen ist. Der Außendurchmesser des Hüllkatheters kann sich von 1,5 bis 4 mm, je nach Anwendungsform, bewegen. In diesem Hüllkatheter 17 ist ein Innenmandrin 20 mit einem Griffstück 19 vorhanden. Bei dem Innenmandrin 20 handelt es sich um einen Draht, welcher aus einem paramagnetischen Werkstoff besteht, und mit einer Kunststoffumhüllung versehen ist. Durch diesen Innenmandrin 20 kann der Hüllkatheter 17 bei Einführung in die Einführschleuse 6 durch die hohe Stabilität des Edelstahldrahtes in den Innenmandrin 20 positionssicher eingeführt werden, und es bestehen weiterhin Steuermöglichkeiten, um die Positionierung des Hüllkatheters 17 durchführen zu können. Auf dem Hüllkatheter 17 sind im oberen Teil gegenüber der Hüllkatheterspitze 21 Markierungen angeordnet, welche in einem 1 cm Abstand vorhanden sind. Dabei ist die erste Markierung zur Hüllkatheterspitze 21 hin so angeordnet, daß bei Einführung in die Einführschleuse 6 und Erreichen der ersten Markierung ca. 30 mm des Hüllkatheters 17 aus der Einführschleuse 6 am distalen Ende hervorragt. Durch Herausnahme des Innenmandrins 20 mit dem Griffstück 19 kann nachfolgend problemlos ein Lichtwellenleiter zur Laserbehandlung des entsprechenden Tumors eingesetzt werden. Aufgrund der erfindungsgemäßen Positionierung des Hüllkatheters 17 durch die Markierung auf dem Hüllkatheter 17 zur Einführschleuse 6 kann zum größten Teil verhindert werden, daß bei Behandlung mit dem Lichtwellenleiter eine Verschmelzung der Lichtquelle mit der lichtundurchlässigen Einführschleuse 6 hervorgerufen wird, da der Hüllkatheter 17 immer ca. 30 mm aus der Einführschleuse 6 am distalen Ende herausragt.

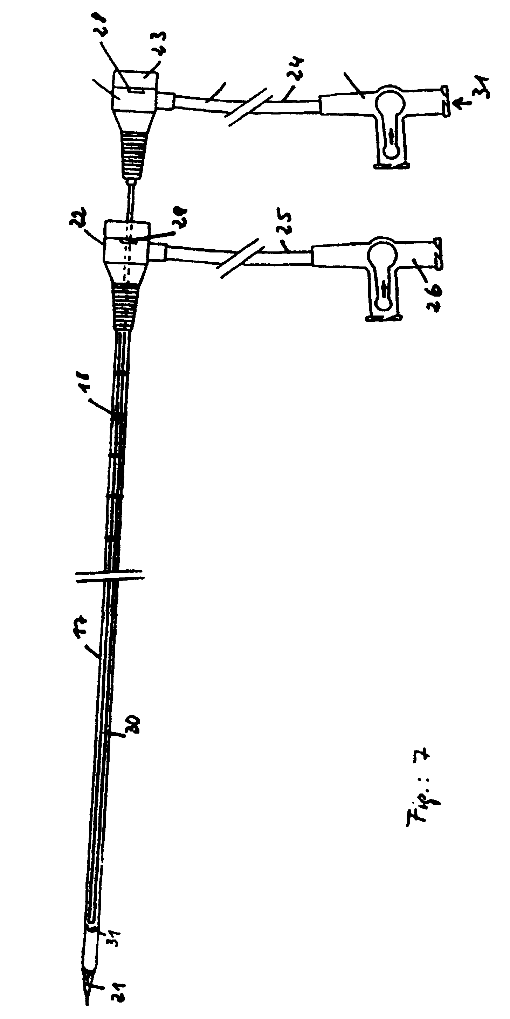

Figur 7 zeigt eine spezielle Ausführungsform des Hüllkatheters mit Kühlung, welcher sich wie folgt zusammensetzt. Dabei wird der Hüllkatheter 17 des Laserapplikationssets in seiner gleichen Art und Weise verwendet, wobei an dem proximalen Ende des Hüllkatheters 17 ein Anschlußstück 22 vorhanden ist. An dem Anschlußstück 22 ist über eine Verbindung 25 ein T-Stück 26 angeschlossen. An diesem T-Stück 26 ist eine Pumpe zur Förderung des Spül- und/oder Kühlmittels vorhanden. Durch ein Verschlußgewinde an dem Anschlußstück 22 wird ein Arretierstück 23 des Innenhüllkatheters 30 fest arretiert. Der Innenhüllkatheter 30 ist innenliegend in dem Hüllkatheter 17. Dabei ist der Innenhüllkatheter 30 so ausgeführt, daß er am distalen Ende 5 mm vor der Hüllkatheterspitze 21 endet. Der Innenhüllkatheter 30 ist am distalen Ende offen. Über dem Arretierstück 23 des Innenhüllkatheters 30 wird über eine Verbindung 24 und einem T-Stück 27 ein Anschluß einer Pumpe für das Spül- und/oder Kühlmittel ebenfalls ausgeführt. Das Kühlmittel 31 gelangt über das T-Stück 24, Verbindung 25, Arretierstück 23 über den Innenhüllkatheter 30 in die Spitze des Hüllkatheters 17 und rückfließend wird das Spül- und/oder Kühlmittel 31 zwischen dem Hüllkatheter 17 und dem Innenhüllkatheter 30 entlang über das Anschlußstück 22, Verbindungsstück 25, T-Stück 26 zur Pumpe geleitet. Dabei wird für das Spül- und/oder Kühlmittel vorzugsweise eine sterile isotone Kochsalzlösung verwendet. In dem Innenhüllkatheter 30 wird der Laserapplikator 33 eingeführt. Somit ist eine maximale Kühlung des gesamten punktierten Gewebes gewährleistet, da durch den Rückfluß zwischen dem Hüllkatheter 17 und dem Innenhüllkatheter 30 die Kühlung auf den gesamten punktierten Gewebebereich übertragen wird, was einen erfindungswesentlichen Vorteil darstellt. Zur Abdichtung bei Einfuhr des Laserapplikators 33 beziehungsweise der Herausnahme der Innenmandrin 20 des Laserapplikationssets ist eine Membrandichtung 28 in dem Arretierstück 23 des Innenhüllkatheters 30 angeordnet. Zur Abdichtung der Einfuhr des Innenhüllkatheters 30 ist in dem Anschlußstück 22 jedenfalls eine Membrandichtung 29 vorhanden.

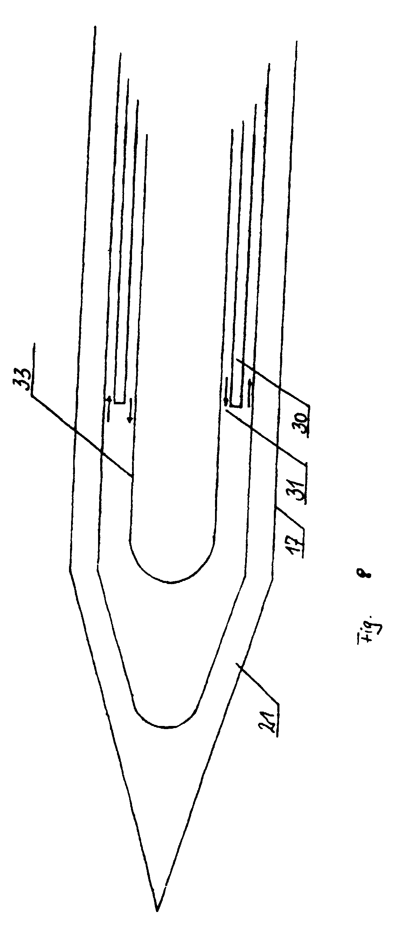

Figur 8 zeigt die Spitze des Hüllkatheters 17 in seiner Ausführungsform. Dabei ist ersichtlich, daß das Kühlmittel 31 im Inneren des Innenhüllkatheters 30 austritt und in der Hüllkatheterspitze 21 an der Außenseite zwischen dem Innenhüllkatheter 30 und dem Hüllkatheter 17 austritt. Da der Laserapplikator 33 über das distale Ende des Innenhüllkatheters 30 herausragt, wird durch den Spül- und/oder Kühlmittelfluß das gesamte äußere Gewebe des zu behandelnden Tumors gekühlt.

Die erfindungsgemäße Anwendung des Laserapplikationssets bei einer Therapiebehandlung von Weichteiltumoren stellt sich wie folgt dar.

Mit der Punktionsnadel 1 und dem beinhalteten Nadelmandrin 2 mit seiner Nadelmandrinspitze 3 wird der zu behandelnde Tumor punktiert. Dabei ist durch die dreikantige Nadelmandrinspitze 3 eine gute Punktionstätigkeit der Punktionsnadel 1 gegeben. Nach erfolgter positiver Punktion wird der Nadelmandrin 2 aus der Punktionsnadel 1 entnommen. Nachfolgend wird bis zum distalen Ende der Punktionsnadel 1 ein Draht 4 eingeführt. Über diesen Draht 4 wird die Punktionsnadel 1 entfernt. Nunmehr wird auf den Draht 4 die Einführschleuse 6 mit arretiertem Mandrin 12 aufgefädelt und bis zum distalen Ende des Drahtes 4 in den zu behandelnden Tumor geleitet. Nach genauer Positionierung der Einführschleuse 6 mit dem Mandrin 12 wird der Draht 4 entnommen. An den Dreiwegehahn 7 werden die notwendigen, je nach Bedarf, zusätzlichen Lokalanästhätika bereitgestellt. Durch eine leichte Drehbewegung des Griffstücks 14 des Mandrins 12 wird die Arretierung mit dem T-Stück 9 der Einführschleuse 6 aufgehoben, und der Mandrin 12 kann aus der Einführschleuse 6 herausgezogen werden.

Eine bevorzugte Ausführung ist weiterhin, daß durch die Markierungen auf der Einführschleuse 6 eine genaue Positionierung der Einführschleuse 6 in den zu behandelnden Tumor durchgeführt werden kann. Nachfolgend wird der Hüllkatheter 17 mit beinhaltetem Innenmandrin 20 in die Einführschleuse 6 eingegeben. Dabei ist anhand der Markierung auf dem Hüllkatheter 17 eine genaue Positionierung des Hüllkatheters 17 in dem zu behandelnden Tumor möglich, wobei erfindungswesentlich hierbei die erste Markierung so ausgeführt ist, daß der Hüllkatheter 17 30 mm am distalen Ende aus der Einführschleuse 6 herausragt. Zur besseren Korrigierbarkeit und Stabilisation bei Einführung des Hüllkatheters 17 ist der Innenmandrin 20 vorhanden. Nach Herausnahme des Innenmandrins 20 mit dem Griffstück 19 wird nun ein Lichtwellenleiter zur entsprechenden Lasertherapie bis zum distalen Ende des Hüllkatheters 17 eingeführt. Durch ständige Überwachung mit Hilfe von CT- bzw. MRT-Verfahren kann eine jeweilige genaue Positionierung des Hüllkatheters 17 mit der erfindungsgemäßen Hüllkatheterspitze 21 durchgeführt werden. Weiterhin kann die Funktionsweise des eingeführten Lichtwellenleiters überprüft werden, um zu verhindern, daß ein Verschmelzen der Lichtquelle mit der nicht lichtdurchlässigen Hülle der Einführschleuse 6 auftritt. Durch die genaue Positionierung mit Hilfe der Einführschleuse 6 und dem Hüllkatheter 17 ist es weiterhin möglich, defekte Lichtwellenleiter zu entnehmen und durch einen neuen Lichtwellenleiter zu ersetzen und somit eine erneute Punktion zu verhindern. Nach erfolgter Behandlung über den Lichtwellenleiter wird dieser entnommen und nachfolgend der Hüllkatheter 17 und die Einführschleuse 6. Dabei ist es möglich, daß zum Beispiel Gewebekleber bei Entnahme der Einführschleuse 6 über den Dreiwegehahn 7 und der Schlauchzuführung 8 an das T-Stück 9 eingeführt wird und somit ein erfindungsgemäßer Vorteil geschaffen ist, indem eine minimale Zellverschleppung des behandelten Tumors bei Herauszug der Einführschleuse 6 stattfindet, was bisher einen wesentlichen Nachteil der Lasertherapie von Tumoren darstellte.

### Bezugszeichen

- 1.: Punktionsnadel
- 2.: Nadelmandrin
- 3.: Nadelmandrinspitze
- 4.: Draht
- 5.: Federende
- 6.: Einführschleuse
- 7.: Dreiwegehahn
- 8.: Schlauchzuführung
- 9.: T-Stück
- 10.: Silikonlamelle
- 11.: Markierung
- 12.: Mandrin
- 13.: Außengewinde
- 13.: 14. Griffstück
- 15.: Mandrinspitze
- 16.: Membran
- 17.: Hüllkatheter
- 18.: Markierung
- 19.: Griffstück
- 20.: Innenmandrin
- 21.: Hüllkatheterspitze
- 22.: Anschlußstück
- 23.: Arretierstück
- 24.: Verbindung
- 25.: Verbindung
- 26.: T-Stück
- 27.: T-Stück
- 28.: Membrandichtung
- 29.: Membrandichtung
- 30.: Innenhüllkatheter
- 31.: Spül- und/oder Kühlflüssigkeit
- 32.: Verschlußgewinde
- 33.: Laserapplikator

## Patentansprüche

1. Laserapplikationsset zur Tumorbehandlung umfassend,
- eine Einführschleuse (6) mit darin arretierbaren innenliegenden, hohlen Mandrin (12) zum Einführen der Einführschleuse entlang einer Punktionsnadel (1) mit Nadelmandrin (2,3,4) in das zu behandelnde Gewebe,
- einen Hüllkatheter (17) aus Kunststoff, der am distalen Ende luft- und flüssigkeitsdicht verschlossen ist und der am proximalen Ende ein Anschlußstück (22) aufweist, wobei der Hüllkatheter (17) nach Entnahme des innenliegenden Mandrins (12) in die Einführschleuse (6) eingeführt werden kann, so daß er über das distale Ende der Einführschleuse (6) hinausragt,
- einen Innenmandrin (20), der in den Hüllkatheter (17) einführbar ist und zu dessen Stabilisation bei der Einführung in und durch die Einführschleuse (6) hindurch bis in das Tumorgewebe dient,
- einen Innenhüllkatheter (30), der, nach Entnahme des Innenmandrins (20) aus dem Hüllkatheter (17), in den Hüllkatheter (17) einführbar ist, wobei der Innenhüllkatheter (30) ein offenes distales Ende sowie ein Arretierstück (23) am proximalen Ende aufweist, über welches mit dem Anschlußstück (22) des Hüllkatheters (17) eine feste, lösbare Verbindung herstellbar ist, so daß der Innenhüllkatheter (30) am distalen Ende 5 mm vor der Hüllkatheterspitze (21) endet,
- einen Lichtwellenleiter (33), der in den Innenhüllkatheter (30) einführbar ist und darin verschiebbar angeordnet ist, so daß sein distales Ende über das distale Ende des Innenhüllkatheters (30) hinausragt,
- wobei der Innenhüllkatheter (30) an seinem distalen Ende einen Kühlmittelaustritt zur Kühlung des distalen Endes des Hüllkatheters (17) aufweist, wodurch eine Kühlung des punktierten Gewebsbereiches und des distalen Endes des Lichtwellenleiters (33) erreicht wird,
- wobei am proximalen Ende der Einführschleuse (6) eine Einrichtung (7,8,9) zur Zuführung von Flüssigkeiten bzw. zur Absaugung von Flüssigkeiten vorhanden ist, und
- wobei sowohl an der Einführschleuse (6) als auch auf dem Hüllkatheter (17) Markierungen vorgesehen sind, so daß eine genaue Positionierung relativ zueinander sowie hinsichtlich des Tumors möglich ist.

2. Laserapplikationsset nach Anspruch 1, **dadurch gekennzeichnet**, daß die Einführschleuse (6) und der Mandrin (12) am distalen und proximalen Ende offen sind.

3. Laserapplikationsset nach Anspruch 1, **dadurch gekennzeichnet**, daß der Hüllkatheter (17) am distalen Ende eine Hüllkatheterspitze (21) aufweist sowie am proximalen Ende offen ist.

4. Laserapplikationsset nach Anspruch 1, **dadurch gekennzeichnet**, daß auf der Einführschleuse (6) und dem Hüllkatheter (17) jeweils Markierungen (18) und (11) so angeordnet sind, damit der Hüllkatheter (17) bei Einschub in die Einführschleuse (6) am distalen Ende der Einführschleuse (6) 30 mm herausragt.

5. Laserapplikationsset nach Anspruch 1 und 4, **dadurch gekennzeichnet**, daß die Markierungen (11, 18) jeweils im Abstand von 1 cm vorhanden sind, wobei auf der Einführschleuse (6) die gesamte Länge markiert ist.

6. Laserapplikationsset nach mindestens einem der vorgenannten Ansprüche, **dadurch gekennzeichnet**, daß die Einführschleuse (6) am proximalen Ende ein T-Stück (9) aufweist, wobei eine Silikonlamelle (10) zwischen T-Stück (9) und Einführschleuse (6) angeordnet ist und an der anderen Seite ein Außengewinde (13) vorhanden ist, mit einer innenliegenden Membran (16), sowie am T-Stück (9) eine Schlauchzuführung (8) mit am Ende vorhandenem Dreiwegehahn (7) ausgeführt ist.

7. Laserapplikationsset nach mindestens einem der vorgenannten Ansprüche, **dadurch gekennzeichnet**, daß ein Griffstück (14) am Mandrin (12) ein Innengewinde aufweist, womit mit dem Außengewinde (13) der Einführschleuse (6) eine Arretierung des Mandrins (12) möglich ist.

8. Laserapplikationsset nach mindestens einem der vorgenannten Ansprüche, **dadurch gekennzeichnet**, daß die Einführschleuse (6) und der Mandrin (12) hohl sind und aus einem Material, welches für das MRT-Verfahren geeignet ist, vorzugsweise PE, FEP, PTFE usw., bestehen.

9. Laserapplikationsset nach mindestens einem der vorgenannten Ansprüche, **dadurch gekennzeichnet**, daß der Mandrin (12) etwa 10 mm am distalen Ende im arretierten Zustand aus der Einführschleuse (6) herausragt und eine offene Mandrinspitze (15) aufweist.

10. Laserapplikationsset nach mindestens einem der vorgenannten Ansprüche, **dadurch gekennzeichnet**, daß der Innenmandrin (20) aus einem Draht, welcher vorzugsweise aus einem paramagnetischen Werkstoff besteht, und mit einer Kunststoffhülle umschlossen ist.

11. Laserapplikationsset nach Anspruch 1, **dadurch gekennzeichnet**, daß in dem Arretierungsstück (23) eine Membrandichtung (28) vorhanden ist und über eine Verbindung (24) ein T-Stück (27) angeschlossen ist.

12. Laserapplikationsset nach Anspruch 1, **dadurch gekennzeichnet**, daß in dem Anschlußstück (22) eine Membrandichtung (29) vorhanden ist und über eine Verbindung (25) ein T-Stück (26) angeschlossen ist.

13. Laserapplikationsset nach Anspruch 11 und 12, **dadurch gekennzeichnet**, daß an dem T-Stück (27 und 26) eine Pumpe zur Förderung des Spül- und/oder Kühlmittels (31) angeschlossen ist.

14. Laserapplikationsset nach Anspruch 13, **dadurch gekennzeichnet**, daß für das Spül- und/oder Kühlmittel (31), welches über den Innenhüllkatheter (30) in den Hüllkatheter (17) gelangt, vorteilhafterweise eine sterile isotone Kochsalzlösung verwendet wird.

15. Laserapplikationsset nach Anspruch 1, **dadurch gekennzeichnet**, daß der Innenhüllkatheter (30) so ausgeführt ist, daß ein Rückfluß des Spül- und/oder Kühlmittels (31) zwischen dem Hüllkatheter (17) und dem Innenhüllkatheter (30) gewährleistet ist.

## Claims

1. A laser application set to treat tumors comprising,
- an introducer tube (6) with internally lockable hollow stylet (12) to insert the introducer tube along a puncture needle (1) with needle-type stylet (2,3,4) into the tissue to be treated,
- a tunneled catheter (17) of plastics, whose distal end is closed in an air- and liquid-tight manner and whose proximal end is provided with a connection piece (22), wherein the tunneled catheter (17), after removal of the internal stylet (12), can be inserted into an introducer tube (6) so that it projects over distal end of the introducer tube (6),
- an internal stylet (20) that can be inserted into the tunneled catheter (17) and is used to stabilize the latter during its insertion into and through the introducer tube (6) into the tumor tissue,
- an inner tunneled catheter (30), that, after the removal of the internal stylet (20) from the tunneled catheter (17), can be inserted into the tunneled catheter (17), wherein the inner tunneled catheter (30) has an open distal end and a locking piece (23) at its proximal end via which a firm, detachable connection can be made with the connection piece (22) of the tunneled catheters (17) so that the inner tunneled catheter (30) ends at its distal end 5 mm before the tip of the tunneled catheter (21),
- a optical waveguide (33) that can be inserted into the inner tunneled catheter (30) in which it is arranged in a movable way so that its distal end projects over the distal end of the inner tunneled catheters (30),
- wherein the inner tunneled catheter (30) has a coolant outlet at its distal end in order to cool the tunneled catheter (17) which facilitates a cooling of the punctured tissue area and the distal end of the optical waveguide (33),
- wherein the proximal end of the introducer tube (6) is provided with an installation (7,8,9) to respectively feed and suck out fluids, and
- wherein both the introducer tube (6) and the tunneled catheter (17) have markings so that they can be exactly positioned in relation to each other and to the tumor.

2. A laser application set according to claim 1, **characterized in that** the introducer tube (6) and the stylet (12) are open at their distal and proximal ends.

3. A laser application set according to claim 1, **characterized in that** the tunneled catheter (17) has a tunneled catheter tip (21) at its distal end is open at its proximal end.

4. A laser application set according to claim 1, **characterized in that** the introducer tube (6) and the tunneled catheter (17) each have markings (18) and (11) that are arranged in such a manner that the tunneled catheter (17), upon insertion into the introducer tube (6) projects 30 mm at the distal end of the introducer tube (6).

5. A laser application set according to claims 1 and 4, **characterized in that** the markings (11, 18) are arranged at a distance of 1 cm from each other and that the entire length of the introducer tube (6) is provided with markings.

6. A laser application set according to at least one of the aforementioned claims, **characterized in that** the introducer tube (6) has a T piece (9) at its proximal end, with a silicone lamella (10) being placed between the T piece (9) and the introducer tube (6) and an external thread (13) fitted to the opposite side, with an internal membrane (16) and a hose lead (8) at the T piece (9) with a three-way faucet (7) fitted to its end.

7. A laser application set according to at least one of the aforementioned claims, **characterized** in that the stylet (12) is equipped with a grip end (14) with an internal thread which, in conjunction with the external thread (13) of the introducer tube (6), enables the locking of the stylet (12).

8. A laser application set according to at least one of the aforementioned claims, **characterized** in that the introducer tube (6) and the stylet (12) are hollow and from a material which is suitable for the MRT process, preferably PE, FEP, PTFE, etc.

9. A laser application set according to at least one of the aforementioned claims, **characterized** in that the stylet (12) in locked state projects by approx. 10 mm from the distal end of the introducer tube (6) and has an open stylet tip (15).

10. A laser application set according to at least one of the aforementioned claims, **characterized** in that the internal stylet (20) is made of a wire, which is preferably from a paramagnetic material, and is enclosed by a synthetic sheathing.

11. A laser application set according to claims 12 and 13, **characterized in that** the locking piece (23) is provided with a membrane sealing (28) and is connected to a T piece (27) by means of a connector (24).

12. A laser application set according to claim 1, **characterized in that** the connection piece (22) is provided with a membrane sealing (29) and connected to a T piece (26) by means of a connector (25).

13. A laser application set according to claims 11 and 12, **characterized in that** a pump to convey a flushing and/or cooling agent (31) is connected to the T piece (27 and 26).

14. A laser application set according to claim 13, **characterized in that** the flushing and/or cooling agent (31), which is conveyed via the inner tunneled catheter (30) into the tunneled catheter (17) is preferably sterile isotonic sodium chloride solution.

15. A laser application set according to claim 1, **characterized in that** the inner tunneled catheter (30) is designed in such a manner that a backflow of the flushing and/or cooling agent (31) is ensured between the tunneled catheter (17) and the inner tunneled catheter (30).

## Revendications

1. Instrumentation de l'application de laser pour le traitement des tumeurs comprenant
- un sas d'insertion (6) avec un mandrin creux, placé à l'interieur (12) pouvant être bloqué dans celui-ci afin d'introduire le sas d'insertion le long d'une aiguille de ponction (1) avec mandrin-aiguille (2,3,4) dans le tissu à traiter,
- un cathéter de gaine (17) en matière synthétique dont le bout périphérique est imperméable à l'air et immersible et dont le bout proximal présente une pièce de raccordement (22) ; le cathéter de gaine (17) pouvant être introduit dans le sas d'insertion (6) après avoir sorti le mandrin placé à l'interieur (12) de manière à ce que le cathéter saillie du bout périphérique du sas d'insertion (6),
- un mandrin interne (20), pouvant être introduit dans le cathéter de gaine (17) et servant à stabiliser celui-ci lors de son introduction dans et à travers le sas d'insertion (6) jusqu'au tissu tumoral,
- un cathéter de gaine interne (30) pouvant être introduit dans le cathéter de gaine (17) après avoir pris le mandrin interne (20) dans le cathéter de gaine (17) ; le cathéter de gaine interne (30) présente un bout périphérique ouvert ainsi qu'une pièce de blocage (23) au bout proximal à l'aide de laquelle un assemblage solide et détachable peut être réalisé avec la pièce de raccordement (22) du cathéter de gaine (17) de manière à ce que le cathéter de gaine interne (30) finisse 5 mm avant la pointe du cathéter de gaine (21) du bout périphérique,
- un guide d'ondes lumineuses (33) pouvant être introduit dans le cathéter de gaine interne (30) où ce guide est disposé de manière mobile de façon à ce que son bout périphérique saillie au bout périphérique du cathéter de gaine interne (30),
- le cathéter de gaine interne (30) présente à son bout périphérique une sortie d'agents refroidisseurs pour le refroidissement du bout périphérique du cathéter de gaine (17) ce qui permet de refroidir les zones du tissu ponctionné et le bout périphérique du guide d'ondes lumineuses (33),
- au bout proximal du sas d'insertion (6) existe un dispositif (7,8,9) pour injecter des liquides ou pour drainer les liquides, et
- sur le sas d'insertion (6) ainsi que sur le cathéter de gaine (17) se trouvent des repères permettant de déterminer la position exacte des éléments et par rapport à la tumeur.

2. Instrumentation d'application de laser selon la revendication 1, **caractérisée en ce** que le sas d'insertion (6) et le mandrin (12) sont ouverts aux bouts périphérique et proximal.

3. Instrumentation d'application de laser selon la revendication 1, **caractérisée en ce** que le cathéter de gaine (17) présente une pointe de cathéter de gaine (21) au bout périphérique et qu'il est ouvert au bout proximal.

4. Instrumentation d'application de laser selon la revendication 1, **caractérisée en ce** que sur le sas d'insertion (6) et sur le cathéter de gaine (17) se trouvent des repères (18) et (11), disposés de manière à permettre au cathéter de gaine (17) saillie de 30 mm du bout périphérique du sas d'insertion (6) quand celui-ci est introduit dans le sas d'insertion (6).

5. Instrumentation d'application de laser selon les revendications 1 et 4, **caractérisée en ce** que les repères (11, 18) sont posés à une distance de 1 cm l'une de l'autre, la longueur totale est indiquée sur le sas d'insertion (6).

6. Instrumentation d'application de laser selon au moins une des revendications citées ci-devant, **caractérisée en ce** que le sas d'insertion (6) dispose d'une pièce en T (9) au bout proximal, une lamelle de silicone (10) est placée entre la pièce en T (9) et le sas d'insertion (6) et l'autre côté présente un filetage (13), avec une membrane située à l'intérieur (16) ; sur la pièce en T (9) est réalisé une arrivée de flexible (8) avec un robinet à trois voies à son bout (7).

7. Instrumentation d'application de laser selon au moins une des revendications citées ci-devant, **caractérisée en ce** qu'une manette (14) du mandrin (12) présente un taraudage permettant de bloquer le mandrin (12) au moyen du filetage (13) du sas d'insertion (6).

8. Instrumentation d'application de laser selon au moins une des revendications citées ci-devant, **caractérisée en ce** que le sas d'insertion (6) et le mandrin (12) sont creux et fabriqués dans un matériau, approprié au procédé de tomographie à résonance magnétique, de préférence en PE, FEP, PTFE, etc.

9. Instrumentation d'application de laser selon au moins une des revendications citées ci-devant, **caractérisée en ce** que le mandrin (12) saillie d'environ 10 mm en position bloquée du sas d'insertion (6) et que celui-ci dispose d'une pointe ouverte (15).

10. Instrumentation d'application de laser selon au moins une des revendications citées ci-devant, **caractérisée en ce** que le mandrin interne (20) est composé d'un fil, réalisé de préférence d'une matière paramagnétique, et entouré d'une gaine en synthétique.

11. Instrumentation d'application de laser selon la revendication 1, **caractérisée en ce** que la pièce de blocage (23) est munie d'un joint à membrane (28) et qu'une pièce en T (27) y est attachée par l'intermédiaire d'un raccord (24).

12. Instrumentation d'application de laser selon la revendication 1, **caractérisée en ce** que la pièce de blocage (22) est munie d'un joint à membrane (29) et qu'une pièce en T (26) y est attachée par l'intermédiaire d'un raccord (25).

13. Instrumentation d'application de laser selon les revendications 11 et 12, **caractérisée en ce** qu'une pompe pour le refoulement de l'agent d'irrigation et/ou de refroidissement (31) est branchée à la pièce en T (27 et 26).

14. Instrumentation d'application de laser selon la revendication 13, **caractérisée en ce** que l'on utilise de préférence une solution d'eau salée isotonique stérile pour l'agent d'irrigation et/ou de refroidissement (31) qui arrive par le cathéter de gaine interne (30) dans le cathéter de gaine (17).

15. Instrumentation d'application de laser selon la revendication 1, **caractérisée en ce** que le cathéter de gaine interne (30) est réalisé de manière à ce qu'un reflux de l'agent d'irrigation et/ou de refroidissement (31) soit assuré entre le cathéter de gaine (17) et le cathéter de gaine interne (30).
